# EUROPÄISCHE PATENTSCHRIFT

(11) **EP 3 821 940 B1**
(45) Veröffentlichungstag und Bekanntmachung des Hinweises auf die Patenterteilung: **01.01.2025**
(21) Anmeldenummer: 19208876.3
(22) Anmeldetag: 13.11.2019
(51) Int. Cl.: A61N 1/05

(54) **ELEKTRODENKONTAKT, ELEKTRODENLEITUNG MIT EINEM ELEKTRODENKONTAKT UND VERFAHREN ZUM ANSCHLIESSEN EINES ELEKTRODENKONTAKTS AN EINE ELEKTRISCHE ZULEITUNG**
ELECTRODE CONTACT, ELECTRODE LEAD COMPRISING AN ELECTRODE CONTACT AND METHOD FOR CONNECTING AN ELECTRODE CONTACT TO AN ELECTRICAL LEAD
CONTACT D'ÉLECTRODE, CONDUIT D'ÉLECTRODE DOTÉ D'UN CONTACT D'ÉLECTRODE ET PROCÉDÉ DE RACCORDEMENT D'UN CONTACT D'ÉLECTRODE À UNE CONDUITE D'ALIMENTATION ÉLECTRIQUE

(43) Veröffentlichungstag der Anmeldung: 19.05.2021
(73) Patentinhaber: BIOTRONIK SE & Co. KG, 12359 Berlin (DE)
(72) Erfinder: Willenberg, Patrick, 12459 Berlin (DE); Kaiser, Dajana, 12247 Berlin (DE); Täubert, Kerstin, 12589 Berlin (DE); Müller, Tobias, 10365 Berlin (DE); Schaarschmidt, Thomas, 15732 Schulzendorf (DE); Eichberg, Roland, 10409 Berlin (DE)
(74) Vertreter: Biotronik Corporate Services SE

(56) Entgegenhaltungen:
- US-A1- 2005 090 885
- US-A1- 2010 179 627
- US-A1- 2010 198 326
- US-A1- 2011 277 324
- US-B1- 7 364 479

## Beschreibung

Die Erfindung betrifft einen Elektrodenkontakt für eine Elektrodenleitung sowie eine Elektrodenleitung für eine Neurostimulationsanwendung, die einen solchen Elektrodenkontakt umfasst. Darüber hinaus betrifft die Erfindung Verfahren zum Anschließen eines solchen Elektrodenkontakts an eine elektrische Zuleitung.

Es ist bekannt, für medizinische Neurostimulationsanwendungen, wie z. B. die Rückenmarkstimulation (englisch spinal cord stimulation - SCS), implantierbare Elektrodenleitungen einzusetzen. Derartige Elektrodenleitungen weisen üblicherweise an einem proximalen Ende einen oder mehrere Stecker zur Kontaktierung eines implantierbaren Pulsgenerators auf. An einem distalen Ende einer derartigen Elektrodenleitung sind ein oder mehrere Elektrodenkontakte zur Kontaktierung eines neuronalen Gewebes vorgesehen. Die Elektrodenkontakte, die auch als Elektrodenpole bezeichnet werden, sind dabei über elektrische Zuleitungen, z. B. in Form von Seilzuleitungen, mit den Steckern am distalen Ende verbunden und können dadurch elektrische Pulse vom Pulsgenerator auf das neuronale Gewebe übertragen. Beispielsweise können mehrere solcher elektrischer Zuleitungen innerhalb eines Elektrodenleitungskörpers verlaufen, der sich zwischen den Steckern und den Elektrodenkontakten von proximal nach distal erstreckt.

Es ist ferner bekannt, dass die Elektrodenkontakte einer solchen Elektrodenleitung an einem sogenannten Paddle der Elektrodenleitung angeordnet sein können. In diesem Fall spricht man auch von einer Paddle-Elektrodenleitung. Das Paddle umfasst einen flach erstreckten (z. B. in etwa "paddelförmigen") elektrisch isolierenden Paddlekörper, der beispielsweise aus einem Kunststoff wie PU und/oder Silikon bestehen kann.

An dem Paddlekörper sind die Elektrodenkontakte in der Weise angeordnet, dass jeweils nur eine in der Anwendung zum Nerv hin weisende Seite des Elektrodenkontakts mit einer Kontaktfläche freiliegt. Die andere Seite ist mitsamt den angebundenen elektrischen Zuleitungen im Isolationsmaterial des Paddlekörpers eingebettet. Beispielsweise kann dabei jeder Elektrodenkontakt mit einer Seilzuleitung mechanisch und elektrisch verbunden sein, wobei die Seilzuleitungen von einem jeweiligen proximalen Stecker in einem oder mehreren (z. B. zwei) Elektrodenleitungskörpern herangeführt werden.

Generell besteht bei einer Elektrodenleitung der beschriebenen Art die Anforderung einer ausreichend sicheren elektrischen und mechanischen Anbindung der elektrischen Zuleitungen sowohl im Steckerbereich als auch insbesondere im Bereich der Elektrodenkontakte (d. h. im Paddlebereich im Fall einer Paddle-Elektrodenleitung).

Eine bekannte Lösung sieht vor, dass zur Kontaktierung eines Elektrodenkontakts eine elektrische Zuleitung auf einer Rückseite eines Elektrodenkontakts, d. h. auf einer der Kontaktfläche gegenüber liegenden Seite des Elektrodenkontakts, angeschweißt wird.

Bei dieser Lösung ist nachteilig, dass die Zuführung und Arretierung der elektrischen Zuleitung bei jedem Elektrodenkontakt jeweils direkt vor dem Schweißprozess erfolgen muss. Dies erfordert einen großen manuellen Aufwand oder eine zusätzliche automatische Vorrichtung. Zudem müssen die elektrischen Zuleitungen im Voraus zugeschnitten sein. Dies erfordert zusätzlichen Platz für Überlängen der elektrischen Zuleitungen, die vorgehalten werden müssen, da sonst die Anordnung unter mechanischer Spannung stehen könnte.

US 2005/090885 A1 offenbart Mittel zur elektrischen Verbindung eines Leiterdrahtes mit einer Elektrode unter Verwendung eines Verbinders (z.B. Crimpröhrchen), der an den Leiter gecrimpt und an den seitlichen Rand der Elektrode geschweißt (z.B. lasergeschweißt) wird. Weiterhin kann ein Crimpanschluss verwendet werden, der ein integraler Bestandteil der Elektrode ist. Der Quetschverbinder kann die Form einer Lasche aufweisen, die gebogen oder gecrimpt werden kann, um den Leiter mit der Elektrode zu verbinden.

Aufgabe der vorliegenden Erfindung ist es, einen verbesserten Elektrodenkontakt für eine Elektrodenleitung vorzuschlagen, der eine sichere elektrische und mechanische Anbindung einer elektrischen Zuleitung erlaubt und zudem die Montage der Elektrodenleitung erleichtert. Ferner sollen eine Elektrodenleitung mit einem solchen Elektrodenkontakt sowie ein Verfahren zum Anschließen eines solchen Elektrodenkontakts an eine elektrische Zuleitung angegeben werden.

Hiervon ausgehend werden die Gegenstände der unabhängigen Ansprüche vorgeschlagen. Merkmale einiger Ausführungsbeispiele sind in den Unteransprüchen angegeben. Die Merkmale der Unteransprüche können miteinander zur Ausbildung weiterer Ausführungsformen kombiniert werden, sofern nicht ausdrücklich etwas anderes angegeben ist.

Gemäß einem ersten Aspekt wird die Aufgabe durch einen Elektrodenkontakt für eine Elektrodenleitung gelöst. Dabei weist der Elektrodenkontakt einen Kontaktkörper mit einer Kontaktfläche zur Kontaktierung von menschlichem oder tierischem neuronalem Gewebe auf. Der Elektrodenkontakt umfasst ferner einen Auslegerabschnitt zur Kontaktierung mindestens einer elektrischen Zuleitung, wobei der Auslegerabschnitt sich von dem Kontaktkörper aus seitlich in einer ersten Raumrichtung, die parallel zu einer Haupterstreckungsebene der Kontaktfläche verläuft, erstreckt, und wobei der Auslegerabschnitt mindestens eine Aussparung zur Hindurchführung der mindestens einen elektrischen Zuleitung aufweist. Erfindungsgemäß ist die mindestens eine Aussparung schlitzförmig ausgebildet. Dabei ist ein Bereich des Auslegerabschnitts durch die schlitzförmige(n) Aussparung(en) in mehrere Stege unterteilt.

Es wird also vorgeschlagen, an einem Kontaktkörper eines Elektrodenkontakts einen seitlichen Auslegerabschnitt vorzusehen. Dieser ermöglicht eine sichere mechanische und elektrische Befestigung bzw. Kontaktierung einer elektrischen Zuleitung an dem Elektrodenkontakt.

Der Auslegerabschnitt ist dabei in dem Sinne ein "seitlicher" Auslegerabschnitt, dass er sich -jedenfalls auch (d. h. jedenfalls teilweise/wenigstens mit einer Richtungskomponente) - in der ersten Raumrichtung erstreckt, die parallel zu der Haupterstreckungsebene der Kontaktfläche verläuft. In einer Draufsicht entlang einer zweiten Raumrichtung, die senkrecht zu der Haupterstreckungsebene verläuft, ragt der Auslegerabschnitt dementsprechend seitlich über den Kontaktkörper hinaus.

Die Kontaktfläche ist zwar dadurch charakterisiert, dass sie eine Haupterstreckungsebene aufweist; abgesehen davon kann sie aber grundsätzlich vielfältige Formen (etwa in einer Draufsicht entlang der zweiten Raumrichtung) und Größen annehmen.

Wenn der Elektrodenkontakt in einem Paddle einer Paddle-Elektrodenleitung angeordnet ist, kann die Haupterstreckungsebene der Kontaktfläche beispielsweise mit einer Paddle-Ebene, d. h. mit einer Haupterstreckungsebene des Paddles, zusammenfallen oder zu dieser parallel verlaufen. Dabei kann die Kontaktfläche freiliegen, um mit Nervengewebe in der Umgebung des Paddles in Kontakt treten zu können. Die übrigen Abschnitte des Elektrodenkontakts, einschließlich des seitlichen Auslegers, können demgegenüber im Isoliermaterial des Paddles eingebettet sein.

Durch den seitlichen Auslegerabschnitt wird dabei eine zusätzliche Hinterschneidung bereitgestellt, die eine bessere (sicherere) Einbettung des Elektrodenkontaktes im Isolationsmaterial des Paddles bewirken kann.

Die seitliche Erstreckung des Auslegerabschnitts hat auch den Vorteil, dass der Elektrodenkontakt dadurch insgesamt sehr flach ausgeführt sein kann. Dies ist insbesondere für eine Verwendung des Elektrodenkontakts bei einer Paddle-Elektrodenleitung günstig, da Paddle, die dabei üblicherweise zum Einsatz kommen, in der Regel nicht dicker als 2 mm sind.

Gemäß einer Ausführungsform ist eine maximale Höhe des Kontaktkörpers in der dritten Raumrichtung senkrecht zu der Haupterstreckungsebene kleiner als eine größte Breite des Kontaktkörpers in der ersten Raumrichtung und/oder als eine größte Breite des Kontaktkörpers in einer zweiten Raumrichtung parallel zu der Haupterstreckungsebene. Dabei kann ein Verhältnis der größten Breite in der dritten Raumrichtung zu der größten Breite in der ersten Raumrichtung und/oder ein Verhältnis der größten Breite in der dritten Raumrichtung zu der größten Breite in der zweiten Raumrichtung beispielsweise kleiner als 0,30 bzw. 0, 15, insbesondere kleiner als 0,25 bzw. 0, 10, bevorzugt kleiner als 0,20 bzw. 0,08 sein.

Die vorstehenden Größenverhältnisse können gemäß einer Variante auch auf den Elektrodenkontakt insgesamt, d. h. insbesondere einschließlich des Auslegerabschnitts, gelten. In dem Fall, dass die größte Breite in der dritten Raumrichtung kleiner als die größte Breite in der ersten Raumrichtung und als die größte Breite in der zweiten Raumrichtung ist, hat der Elektrodenkontakt folglich eine flache Grundform. Dies ist z. B. für eine Anordnung an einem flachen Paddle einer Paddle-Elektrodenleitung vorteilhaft, wie bereits erläutert. Dadurch, dass der seitliche Auslegerabschnitt eine Aussparung zur Hindurchführung einer elektrischen Zuleitung aufweist, wird eine - ggf. vorläufige - mechanische Fixierung der Zuleitung an dem Elektrodenkontakt ermöglicht. Dies erlaubt beispielsweise, eine Vormontage mit einer mechanischen Arretierung für einen nachgelagerten Schweißprozess, in welchem die Zuleitung stoffschlüssig und elektrisch leitend mit dem Elektrodenkontakt verbunden wird. Dadurch wird vor dem Schweißen eine genaue Positionierung in entspannter Lage der Zuleitung möglich, ohne dass die Zuleitung bereits im Voraus auf eine präzise Länge zugeschnitten werden muss. Um Montagetoleranzen auszugleichen, ist es nämlich hilfreich, wenn die Länge der Zuleitung während der Montage der tatsächlichen Elektrodenleitungslänge angepasst werden kann. Mit anderen Worten kann z. B. eine Zuleitung an einem erfindungsgemäßen Elektrodenkontakt einer Paddle-Elektrodenleitung in richtiger Position und in entspannter Lage mechanisch arretiert werden, so dass der Schweißprozess und der Zuschnitt zu einem späteren Zeitpunkt durchgeführt werden kann.

Ein weiterer Vorteil des erfindungsgemäßen Elektrodenkontakts besteht im Zusammenhang mit der Montage einer Elektrodenleitung darin, dass eine Zuführung der elektrischen Zuleitung zu dem Elektrodenkontakt nicht unmittelbar vor dem Schweißprozess erfolgen muss, sondern bereits zuvor - z. B. an einem anderen Fertigungsort - stattfinden kann. Zudem muss die Zuleitung während des Schweißprozesses nicht manuell oder mit einer gesonderten Vorrichtung gehalten werden. Damit ist ein gut reproduzierbarer Prozess mit reduziertem Mitarbeitereinfluss möglich.

Der seitliche Auslegerabschnitt ermöglicht es z. B., dass bei einem Widerstandsschweißprozess die Schweißelektroden ungehindert durch eine Freimachung des vorgefertigten Paddles beidseitig an die jeweilige Schweißfläche herangeführt werden. Bei einem Paddle mit zumeist mehreren Elektrodenkontakten (z. B. 16 Stück) können so alle Verbindungen zunächst mechanisch arretiert und im Anschluss daran direkt nacheinander elektrisch kontaktiert werden.

Bei einer bevorzugten Ausführungsform ist der Auslegerabschnitt ausgebildet, die mindestens eine elektrische Zuleitung kraftschlüssig zu fixieren, wenn sie durch die mindestens eine Aussparung hindurch geführt ist. Beispielsweise kann die Zuleitung durch ihre Spannung und/oder unter aktiver Einwirkung des (z. B. elastisch verformbaren) Auslegerabschnitts in der Aussparung geklemmt werden.

Es liegt ferner im Rahmen der Erfindung, dass der Auslegerabschnitt mindestens eine Befestigungsfläche aufweisen kann, auf der ein Abschnitt der mindestens einen elektrischen Zuleitung zu liegen kommt, wenn die Zuleitung durch die mindestens eine Aussparung hindurch geführt ist. Dabei kann insbesondere vorgesehen sein, dass die mindestens eine Befestigungsfläche für eine stoffschlüssige Befestigung des Abschnitts der mindestens einen elektrischen Zuleitung, insbesondere mittels Schweißen, geeignet ist. Mit anderen Worten handelt es sich bevorzugt um eine schweißbare Befestigungsfläche, die auch als Schweißbereich bezeichnet werden kann.

Bei einer Ausführungsform weist der Auslegerabschnitt mehrere, wie z. B. mindestens zwei, derartige Aussparungen auf.

Eine durch eine oder mehrere schlitzförmige Aussparung(en) segmentierte Befestigungsfläche des Elektrodenkontaktes am Paddle kann die aus einem oder mehreren Elektrodenleitungskörper(n) zugeführte(n) Zuleitung(en) aufnehmen. Dabei wird z. B. eine Zuleitung durch seitliche Schlitze in dem Auslegerabschnitt, die die Aussparungen bilden, im Wechsel (oben/unten) eingeführt. Durch eine entsprechend angepasste Breite des Schlitzes bzw. der Schlitze wird die Zuleitung geklemmt oder/und durch die erzeugte Eigenspannung in Position gehalten. Anschließend kann durch einen Schweißprozess eine elektrische Kontaktierung erfolgen.

In einer anderen Variante ist die mindestens eine Aussparung lochförmig. Beispielsweise kann die mindestens eine Aussparung in Form einer oder mehrerer Bohrungen und/oder eines oder mehrerer Durchbrüche vorgesehen sein.

Dabei können bei beiden vorgenannten Varianten die Schlitzbreiten bzw. Lochdurchmesser mit Blick auf einen Durchmesser der verwendeten elektrischen Zuleitung gezielt so gewählt sein, dass sich eine gute mechanische Arretierung ergibt.

Bei einer weiteren Ausführungsvariante weist der Auslegerabschnitt eine oder mehrere Vertiefungen oder Kröpfungen auf, die ein seitliches Wegrutschen der elektrischen Zuleitung verhindern.

Es kann ferner vorgesehen sein, dass die mindestens eine Aussparung schlitzförmig ist und einen Bereich des Auslegerabschnitts in mehrere Stege unterteilt, wobei wenigstens ein Steg gegenüber wenigstens einem anderen Steg eine Schrägstellung (z. B. um einen Winkel im Bereich von 10° bis 30°) aufweist.

Gemäß einem zweiten Aspekt wird eine Elektrodenleitung für eine Neurostimulationsanwendung vorgeschlagen. Die Elektrodenleitung umfasst ein flaches Paddle mit einer Anzahl von Elektrodenkontakten zur Kontaktierung von menschlichem oder tierischem neuronalem Gewebe. Dabei umfasst die Anzahl von Elektrodenkontakten mindestens einen Elektrodenkontakt nach gemäß dem ersten Erfindungsaspekt.

Die Elektrodenleitung umfasst bei einer Ausführungsform mindestens eine elektrische Zuleitung, die an dem Auslegerabschnitt des mindestens einen Elektrodenkontakts befestigt ist. Beispielsweise kann die mindestens eine elektrische Zuleitung an dem Auslegerabschnitt des mindestens einen Elektrodenkontakts stoffschlüssig, insbesondere mittels Schweißen, befestigt sein.

Gemäß einer Ausführungsform umfasst die Elektrodenleitung mindestens eine elektrische Zuleitung, welche den Auslegerabschnitt des mindestens einen Elektrodenkontakts elektrisch kontaktiert.

Ein dritter Aspekt betrifft ein Verfahren zum Anschließen mindestens eines Elektrodenkontakts an mindestens eine elektrische Zuleitung. Bei dem Elektrodenkontakt kann es sich insbesondere um einen Elektrodenkontakt einer Elektrodenleitung für eine Neurostimulationsanwendung handeln. Das Verfahren umfasst das Bereitstellen mindestens eines Elektrodenkontakts gemäß dem ersten Erfindungsaspekt und das Befestigen der mindestens einen elektrischen Zuleitung an dem Auslegerabschnitt des mindestens einen Elektrodenkontakts in der Weise, dass die elektrische Zuleitung sich durch die mindestens eine Aussparung hindurch erstreckt.

Die mindestens eine elektrische Zuleitung kann gemäß einer Ausführungsform stoffschlüssig, insbesondere mittels Schweißen, an dem Auslegerabschnitt befestigt werden.

Beispielsweise kann gemäß einer Ausführungsform die mindestens eine Aussparung schlitzförmig sein und dadurch einen Bereich des Auslegerabschnitts in mehrere Stege unterteilen. Dabei kann im Rahmen des erfindungsgemäßen Verfahrens ein Abschnitt der elektrischen Zuleitung in die mindestens eine schlitzförmige Aussparung eingelegt werden. Dieser Abschnitt kann sodann durch Verformen mindestens eines der Stege eingeklemmt werden. Mit anderen Worten kann mindestens ein Steg derart verbogen werden, dass er die elektrische Zuleitung an dem Auslegerabschnitt festklemmt. Dabei kann der mindestens eine Steg z. B. plastisch verformt werden.

Bei einer Variante dieser Ausführungsform kann durch eine ausreichende mechanische und elektrische Kontaktierung der Zuleitung in Folge des Verformens eines oder mehrerer Stege ein nachgelagerter Schweißprozess ganz entfallen.

Bei einer weiteren Variante des erfindungsgemäßen Verfahrens werden mehrere Elektrodenkontakte gemäß dem ersten Erfindungsaspekt an eine oder mehrere elektrische Zuleitungen angeschlossen. Dabei werden die elektrische Zuleitung oder die elektrischen Zuleitungen zunächst vorläufig an den jeweiligen Auslegerabschnitten der Elektrodenkontakte befestigt, derart, dass sie sich durch die Aussparungen in den Auslegerabschnitten hindurch erstrecken. Optional kann sodann eine Positionierung einer oder mehrerer der elektrischen Zuleitungen relativ zu den Auslegerabschnitten angepasst werden. Danach werden die elektrische Zuleitung oder die elektrischen Zuleitungen, insbesondere mittels Schweißen, in einer jeweiligen finalen Position an den Auslegerabschnitten befestigt.

Eine Weiterbildung dieser Variante sieht vor, dass sich ein weiterer Verfahrensschritt anschließt, in welchem die elektrische Zuleitung oder die elektrischen Zuleitungen zugeschnitten werden.

Weitere Vorteile und Ausführungsformen der vorliegenden Erfindung sollen nachfolgend mit Bezug auf die Figuren beschrieben werden. Es zeigen:
- Fig. 1: schematisch und beispielhaft einen distalen Endabschnitt einer Elektrodenleitung für eine Neurostimulationsanwendung gemäß einer oder mehrerer Ausführungsformen;
- Fig. 2: schematisch und beispielhaft einen Elektrodenkontakt gemäß einer oder mehrerer Ausführungsformen;
- Fig. 3: schematisch und beispielhaft einen Elektrodenkontakt gemäß einer oder mehrerer Ausführungsformen;
- Fig.4A-B: schematisch und beispielhaft jeweils einen Elektrodenkontakt gemäß einer oder mehrerer Ausführungsformen;
- Fig.5A-B: schematisch und beispielhaft jeweils einen Elektrodenkontakt gemäß einer oder mehrerer Ausführungsformen;
- Fig.6A-B: schematisch und beispielhaft jeweils einen Elektrodenkontakt gemäß einer oder mehrerer Ausführungsformen;
- Fig.7A-B: schematisch und beispielhaft jeweils Schritte eines Verfahrens zum Anschließen eines Elektrodenkontakts an eine elektrische Zuleitung gemäß einer oder mehrerer Ausführungsformen;
- Fig.8A-B: schematisch und beispielhaft jeweils Schritte eines Verfahrens zum Anschließen eines Elektrodenkontakts an eine elektrische Zuleitung gemäß einer oder mehrerer Ausführungsformen;
- Fig.9A-B: schematisch und beispielhaft jeweils einen Elektrodenkontakt gemäß einer oder mehrerer Ausführungsformen;
- Fig. 10: schematisch und beispielhaft einen Elektrodenkontakt gemäß einer oder mehrerer Ausführungsformen, welcher an zwei elektrischen Zuleitungen angeschlossen ist; und
- Fig. 11: schematisch und beispielhaft zwei Elektrodenkontakte gemäß einer oder mehrerer Ausführungsformen, welche an eine gemeinsame elektrische Zuleitung angeschlossen sind.

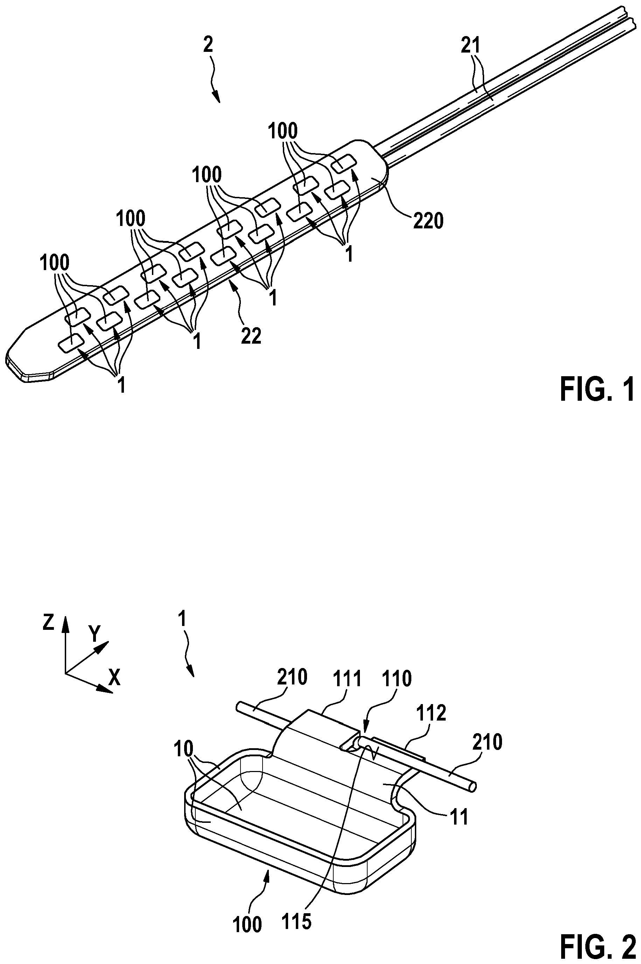

Fig. 1 zeigt schematisch und beispielhaft einen distalen Endabschnitt einer Elektrodenleitung 2 für eine Neurostimulationsanwendung gemäß einer oder mehrerer Ausführungsformen.

Die Elektrodenleitung 2 umfasst ein flaches Paddle 22 mit mehreren Elektrodenkontakten 1, die zur elektrischen Kontaktierung von menschlichem oder tierischem neuronalem Gewebe dienen.

Die Elektrodenkontakte 1, werden durch eine oder mehrere (in Fig. 1 nicht einzeln dargestellte) elektrische Zuleitungen 210, etwa in Form von Seilzuleitungen, kontaktiert, die in zwei Elektrodenleitungskörpern 21 von proximal nach distal an das Paddle 22 herangeführt werden.

Das Paddle 22 umfasst einen flach erstreckten, elektrisch isolierenden Paddlekörper 220, der beispielsweise aus einem Kunststoff wie PU und/oder Silikon bestehen kann. Bevorzugt weist das Paddle eine Dicke von maximal 2 mm auf.

An dem Paddlekörper 220 sind die Elektrodenkontakte 1 in der Weise angeordnet, dass jeweils nur eine in der Anwendung zum Nerv hin weisende Seite des Elektrodenkontakts 1 mit einer Kontaktfläche 100 freiliegt. Bei dem gezeigten Ausführungsbeispiel sind die Kontaktflächen im Wesentlichen rechteckig und eben, wobei die Dimensionen beispielsweise 3,5 mm x 2,0 mm betragen können. Die andere Seite des jeweiligen Elektrodenkontakts 1 ist mitsamt den angebundenen elektrischen Zuleitungen 210 im Isolationsmaterial des Paddlekörpers 220 eingebettet und in Fig. 1 dementsprechend nicht sichtbar.

Fig. 2 zeigt schematisch und beispielhaft einen derartigen Elektrodenkontakt 1 gemäß einer oder mehrerer Ausführungsformen. In der perspektivischen Darstellung gemäß Fig. 2 ist insbesondere die Rückseite des Elektrodenkontakts 1 gezeigt, die in der Darstellung in Fig. 1 nicht sichtbar ist, da sie dort in den Paddlekörper 220 eingebettet ist. Die in Fig. 1 dargestellte Kontaktfläche 100 weist in der Darstellung gemäß Fig. 2 nach unten und ist dort als solche nicht sichtbar.

Das in Fig. 2 eingezeichnete Koordinatensystem verdeutlicht, dass die Kontaktfläche 100 sich im Wesentlichen entlang einer Haupterstreckungsebene XY erstreckt. Dabei wird die Kontaktfläche 100 durch die ebene Unterseite eines wannenförmigen Kontaktkörpers 10 gebildet.

Zur Kontaktierung einer elektrischen Zuleitung 210 umfasst der Elektrodenkontakt 1 einen seitlichen Auslegerabschnitt 11, der sich von dem Kontaktkörper 10 aus seitlich in einer ersten Raumrichtung Y erstreckt. Dabei verläuft die erste Raumrichtung Y parallel zu der Haupterstreckungsebene XY der Kontaktfläche 100.

Der Elektrodenkontakt 1, einschließlich des seitlichen Auslegerabschnitts 11, ist insgesamt vergleichsweise flach ausgebildet. Insbesondere ist eine maximale Höhe des Elektrodenkontakts 1 in einer dritten Raumrichtung Z, die senkrecht zu der Haupterstreckungsebene XY verläuft, deutlich kleiner als eine jeweilige maximale Breite des Elektrodenkontakts 1 in der ersten Raumrichtung Y und in einer zweiten Raumrichtung X parallel zu der Haupterstreckungsebene XY.

Der Elektrodenkontakt kann beispielsweise aus einer Platin-Iridium-Legierung (Pt/Ir) bestehen oder ein solches Material umfassen.

Die elektrische Zuleitung 210 kann beispielsweise eine isolierte Seilzuleitung, wie etwa ein DFT-Draht (MP35N / Ag), sein. Dabei kann ein Außendurchmesser der Seilzuleitung 210 z. B. 0,14 mm betragen.

Zur Befestigung der elektrischen Zuleitung 210, die bei dem vorliegenden Ausführungsbeispiel eine isolierte Seilzuleitung ist, weist der Auslegerabschnitt 11 seitlich eine schlitzförmige Aussparung 110 auf. Der so gebildete Schlitz 110 kann beispielsweise eine Breite von ca. 0,3 mm bis 0,5 mm haben.

Die schlitzförmige Aussparung 110 unterteilt einen seitlichen Bereich des Auslegerabschnitts 11 in einen ersten Steg 111 und einen zweiten Steg 112. Die elektrische Zuleitung 210 ist über den zweiten Steg 112 hinweg durch den Schlitz 110 nach unten geführt und verläuft unter dem ersten Steg 111 hindurch. Die Zuleitung 210 verspannt sich in dem Schlitz 110 und ist dadurch arretiert. Sie kann zusätzlich auf eine schweißbare Befestigungsfläche 115, die durch eine Oberseite des zweiten Stegs 112 gebildet wird, geschweißt und ggf. anschließend hinter dem ersten Steg 111 zugeschnitten werden. Auf diese Weise wird eine sichere und passgenaue mechanische Anbindung und elektrische Kontaktierung der Zuleitung 210 an dem Elektrodenkontakt 1 ermöglicht.

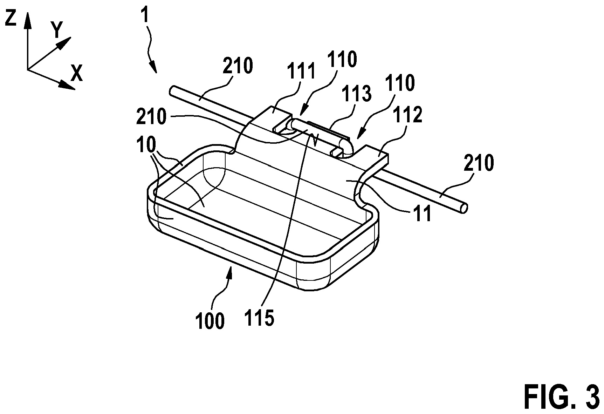

Die Fig. 3 zeigt eine Ausführungsvariante, bei der in dem Auslegerabschnitt 11 zwei derartige Schlitze 110 ausgebildet sind, sodass der seitliche Bereich des Auslegerabschnitts 11 in einen ersten Steg 111, einen zweiten Steg 112 und einen (mittleren) dritten Steg 113 unterteilt ist. Dabei wird die Zuleitung 210 von unten durch einen der Schlitze 110 nach oben über den dritten Steg 113 und durch den anderen Schlitz 110 wieder nach unten geführt. Die Zuleitung 210 verspannt sich in dieser Wechselanordnung und ist dadurch arretiert. Sie kann später auf die durch die Oberseite des dritten Stegs 113 gebildete Befestigungsfläche 115 geschweißt werden und optional hinter dem ersten Steg 111 auf Länge zugeschnitten werden.

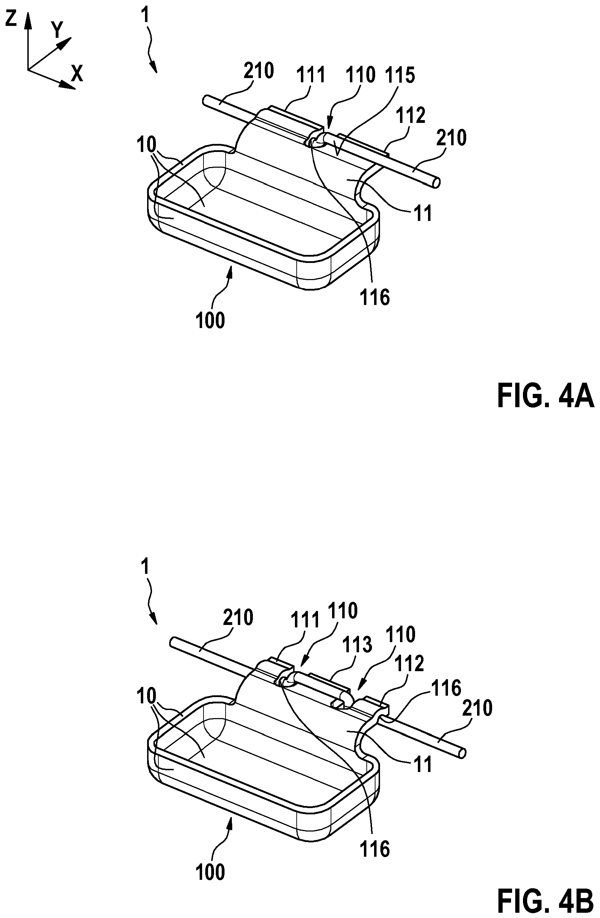

Die in Fig. 4A und 4B dargestellten Elektrodenkontakte 1 entsprechen weitgehend den Ausführungsbeispielen gemäß Fig. 2 bzw. Fig. 3, wobei jedoch jeweils zusätzlich Vertiefungen 116 oder Kröpfungen in den durch die Schlitze 110 entstandenen Stegen 111, 112, 113 ausgebildet sind, die ein seitliches Wegrutschen der elektrischen Zuleitung 210 verhindern.

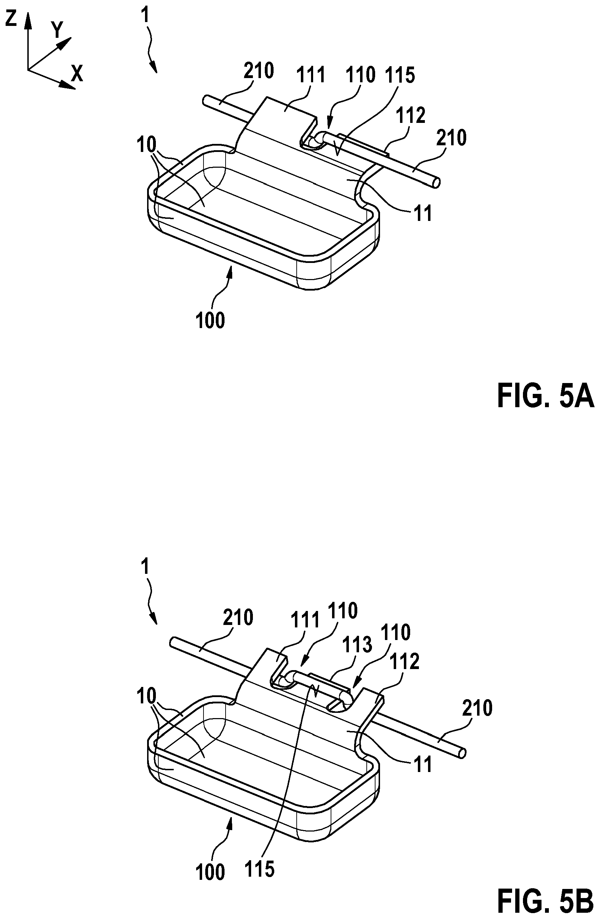

Auch die in den Fig. 5A und 5B gezeigten Elektrodenkontakte 1 entsprechen weitgehend den Ausführungsbeispielen gemäß Fig. 2 bzw. Fig. 3, mit dem Unterschied, dass der erste Steg 111 gegenüber dem zweiten Steg 112 (in Fig. 5A) bzw. der erste Steg 111 und der zweite Steg 112 gegenüber dem dritten Steg 113 (in Fig. 5B) eine Schrägstellung (z. B. um einen Winkel im Bereich von 10° bis 30°) aufweisen, wodurch ein gerades Durchlaufen der Seilzuleitung 210 ermöglicht wird. Beispielsweise kann, wie in den Fig. 5A-B dargestellt, ein Steg 112, 113 im Wesentlichen parallel zu der Haupterstreckungsebene XY ausgerichtet sein, wohingegen der andere Steg 111 bzw. die anderen Stege 111, 112 - z. B. um 10° bis 30° - gegenüber der Haupterstreckungsebene XY abgewinkelt sind.

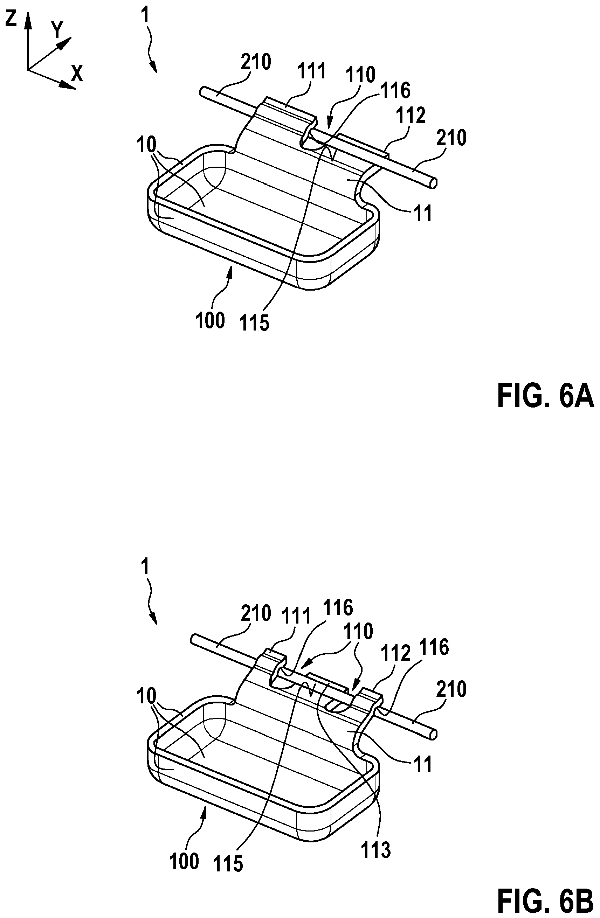

Die Elektrodenkontakte 1 gemäß den Fig. 6A und 6B gehen jeweils aus einer Kombination der Ausführungsvarianten gemäß Fig. 4A und Fig. 5A bzw. gemäß Fig. 4B und Fig. 5B hervor. Damit ist gemeint, dass eine Vertiefung 116 bzw. Kröpfung des Auslegerabschnitts 11 entsprechend Fig. 4A-B mit einer Schrägstellung eines oder mehrerer Stege 111, 112 entsprechend Fig. 5A-B kombiniert ist.

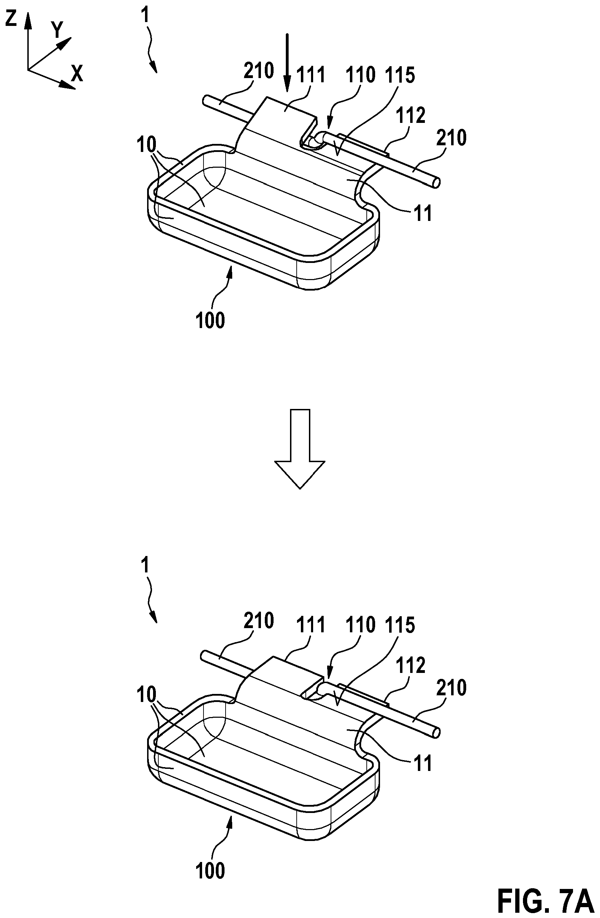

Die Fig. 7A-8B verdeutlichen jeweils eine Ausführungsvariante des erfindungsgemäßen Verfahrens zum Anschließen eines Elektrodenkontakts 1 an eine elektrische Zuleitung 210.

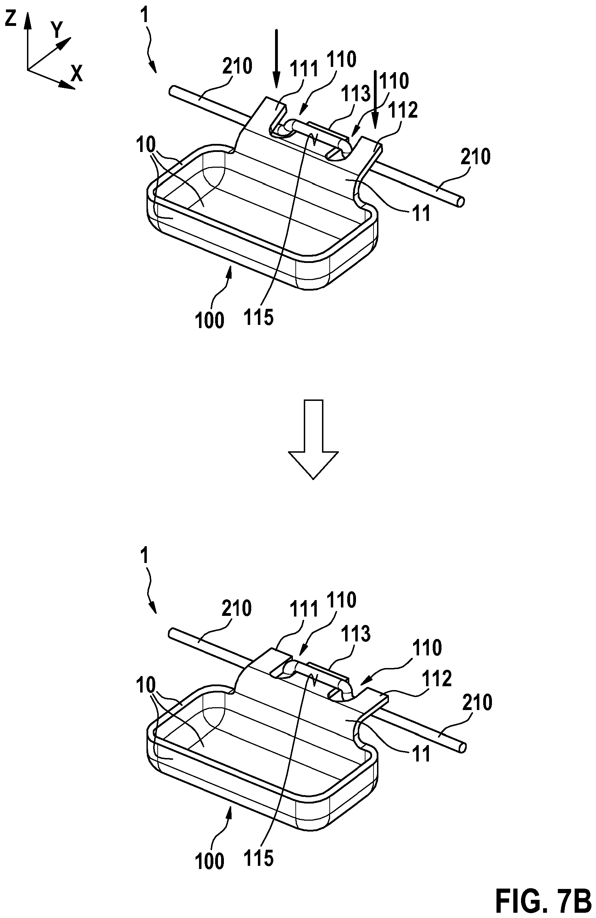

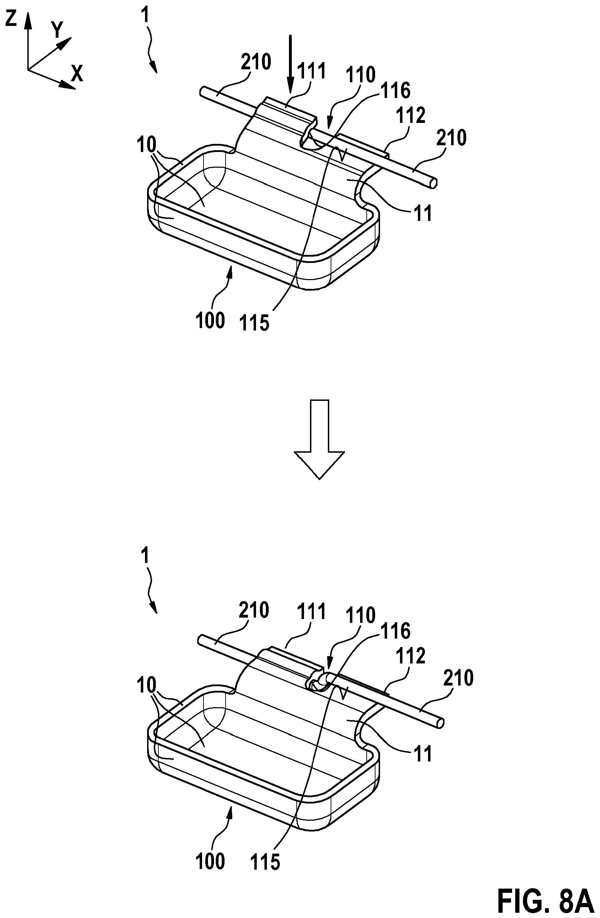

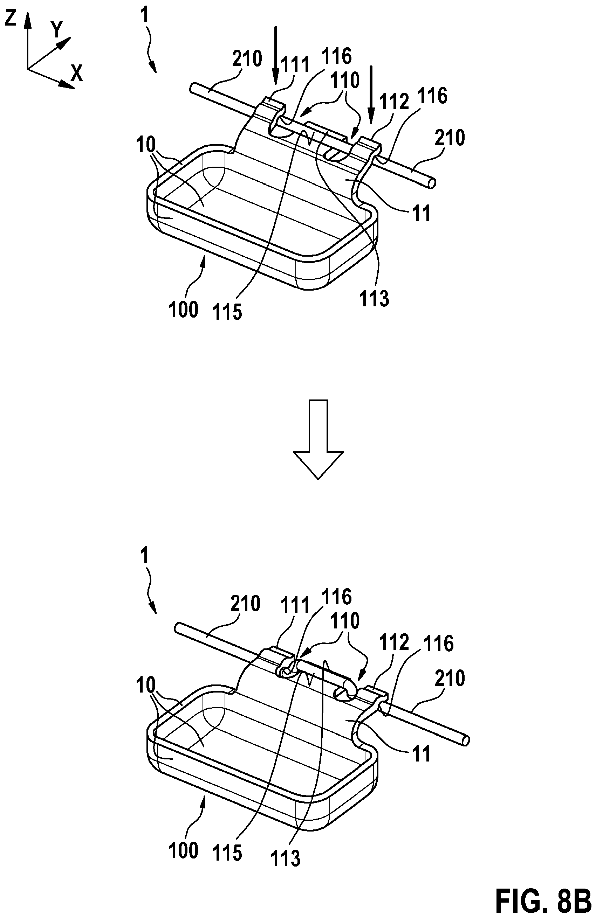

Dabei weist der jeweilige Auslegerabschnitt 11 bei den Ausführungsbeispielen gemäß Fig. 7A und 8A eine einzige schlitzförmige Aussparung 110, wodurch zwei Stege 111, 112 definiert werden. Demgegenüber weist der jeweilige Auslegerabschnitt 11 bei den Ausführungsbeispielen gemäß Fig. 7B und 8B zwei schlitzförmige Aussparungen 110 auf, sodass jeweils drei Stege 111, 112, 113 entstehen. Bei den Auslegerabschnitten 11 gemäß Fig. 8A-B ist zusätzlich eine Vertiefung 116 vorgesehen.

In einem j eweiligen oberen Bildteil der Fig. 7A-8B ist veranschaulicht, dass zum Befestigen der elektrischen Zuleitung 210 zunächst ein Abschnitt der Zuleitung 210 in die schlitzförmige(n) Aussparung(en) 110 eingelegt wird. Dabei sind zunächst jeweils ein oder mehrere der Stege 111, 112 gegenüber den übrigen Stegen 112, 113 schräggestellt. Dadurch kann die Zuleitung 210 leicht in die j eweilige(n) Aussparung(en) eingeführt werden.

Der Abschnitt der Zuleitung 210 wird sodann durch Verformen mindestens eines der Stege 111, 112 zwischen den Stegen 111, 112, 113 eingeklemmt, wie in einem jeweiligen unteren Bildteil der Fig. 7A-8B gezeigt. Mit anderen Worten wird mindestens ein Steg 111, 112 derart verbogen, dass er die elektrische Zuleitung 210 an dem Auslegerabschnitt 11 festklemmt. Dabei kann der mindestens eine Steg 111, 112 z. B. plastisch verformt werden.

Bei einer Variante dieser Ausführungsform kann durch eine ausreichende mechanische und elektrische Kontaktierung der Zuleitung 210 in Folge des Verformens des oder der Stege 111, 112 ein nachgelagerte Schweißprozess ganz entfallen.

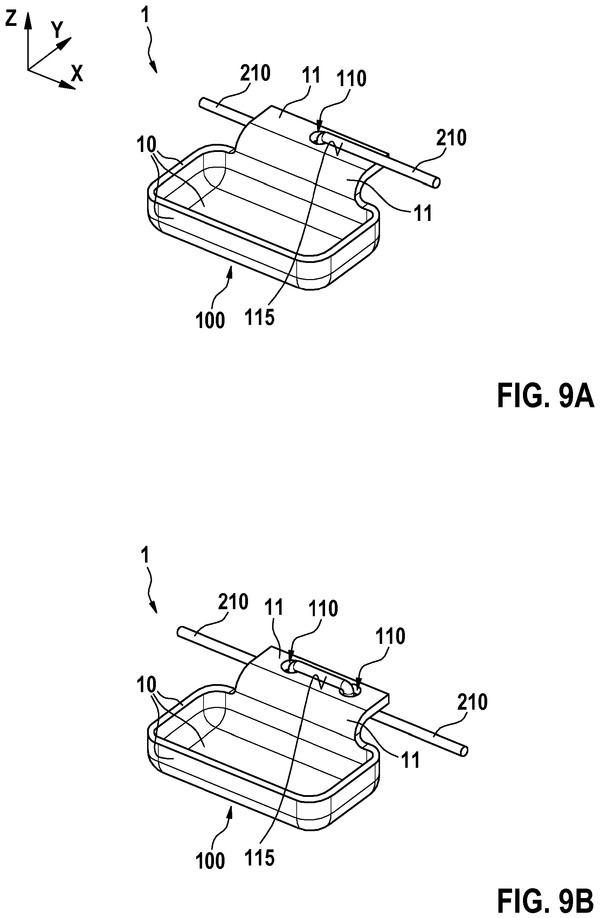

Die in Fig. 9A und 9B dargestellten Elektrodenkontakte 1 entsprechen prinzipiell den weiter oben erläuterten Ausführungsbeispielen gemäß Fig. 2 bzw. Fig. 3, mit dem Unterschied, dass die Aussparungen 110 nicht schlitzförmig, sondern lochförmig sind. Mit anderen Worten sind die Schlitze 110 hier durch Bohrungen oder Durchbrüche 110 ersetzt. Dementsprechend wird die elektrische Zuleitung 210 bei diesen Ausführungsformen nicht von der Seite eingelegt, sondern direkt durch die lochförmigen Aussparungen 110 geführt.

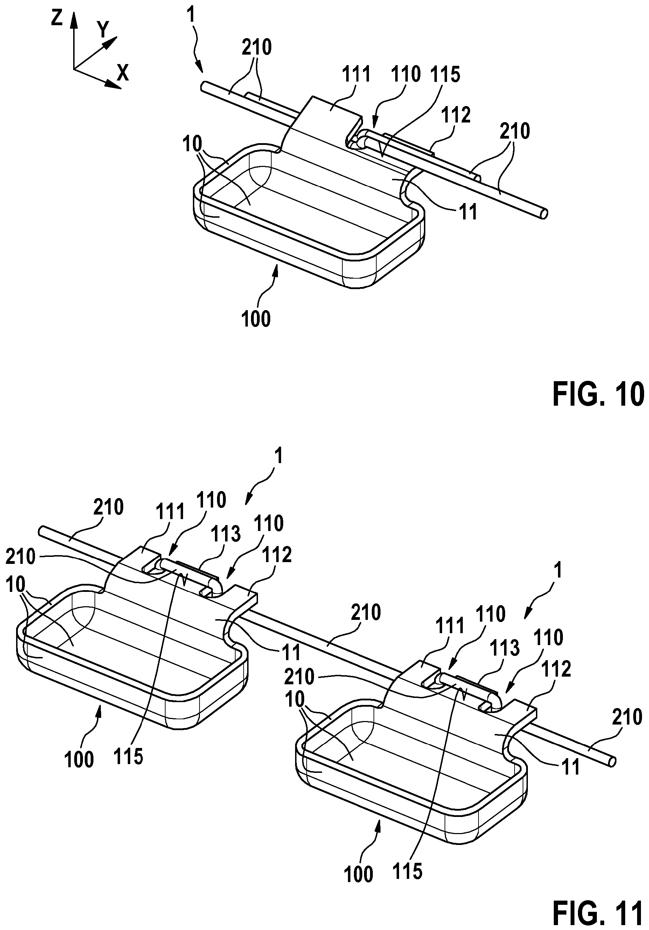

Die Fig. 10 veranschaulicht eine Ausführungsform, bei welcher zwei parallele Zuleitungen 210 in der vorstehend beschriebenen Weise an dem seitlichen Auslegerabschnitt 11 eines Elektrodenkontakts 1 befestigt sind. Grundsätzlich können auch mehr als zwei parallele Zuleitungen 210 vorgesehen sein, wobei die Stege 111, 112 bzw. der Schlitz 110, durch welchen die Zuleitungen 210 hindurch geführt werden, nach Bedarf in der ersten Raumrichtung Y verlängert werden können, um ausreichend Platz für die mehreren Zuleitungen 210 zu bieten.

Selbstverständlich kann die Zuleitungsführung auch über mehrere Elektrodenkontakte 1 hinweg fortgesetzt werden, in der Weise, dass eine Zuleitung 210 sequenziell an mehrere Elektrodenkontakte 1 angeschlossen wird. In Fig. 11 ist dies beispielsweise mit einer Zuleitung 210 und zwei daran angeschlossenen Elektrodenkontakten 1 veranschaulicht.

### Bezugszeichenliste

- 1: Elektrodenkontakt
- 10: Kontaktkörper
- 100: Kontaktfläche

- 11: Auslegerabschnitt
- 110: Aussparung
- 111: Erster Steg
- 112: Zweiter Steg
- 113: Dritter Steg
- 115: Befestigungsfläche
- 116: Vertiefung

- 2: Elektrodenleitung
- 21: Elektrodenleitungskörper
- 210: Elektrische Zuleitung
- 22: Paddle
- 220: Paddlekörper

- X: Zweite Raumrichtung
- Y: Erste Raumrichtung
- Z: Dritte Raumrichtung

- XY: Haupterstreckungsebene

## Patentansprüche

1. Elektrodenkontakt (1) für eine Elektrodenleitung (2) zur Neurostimulation, wobei der Elektrodenkontakt (1) einen Kontaktkörper (10) mit einer Kontaktfläche (100) zur Kontaktierung von menschlichem oder tierischem neuronalem Gewebe aufweist, sowie
einen Auslegerabschnitt (11) zur Kontaktierung mindestens einer elektrischen Zuleitung (210), wobei der Auslegerabschnitt (11) sich von dem Kontaktkörper (10) aus seitlich in einer ersten Raumrichtung (Y), die parallel zu einer Haupterstreckungsebene (XY) der Kontaktfläche (100) verläuft, erstreckt, und wobei der Auslegerabschnitt (11) mindestens eine Aussparung (110) zur Hindurchführung der mindestens einen elektrischen Zuleitung (210) aufweist,
**dadurch gekennzeichnet,**
**dass** die mindestens eine Aussparung (110) schlitzförmig ist und ein Bereich des Auslegerabschnitts (11) durch die schlitzförmige Aussparung (110) in mehrere Stege unterteilt ist.

2. Elektrodenkontakt (1) nach Anspruch 1, **dadurch gekennzeichnet, dass** eine maximale Höhe des Kontaktkörpers (100) in einer dritten Raumrichtung (Z) senkrecht zu der Haupterstreckungsebene (XY) kleiner ist als
- eine größte Breite des Kontaktkörpers (100) in der ersten Raumrichtung (Y) und/oder
- eine größte Breite des Kontaktkörpers (100) in einer zweiten Raumrichtung (X) parallel zu der Haupterstreckungsebene (XY).

3. Elektrodenkontakt (1) nach einem der vorstehenden Ansprüche, **dadurch gekennzeichnet, dass** der Auslegerabschnitt (11) ausgebildet ist, die mindestens eine elektrische Zuleitung (210) kraftschlüssig zu fixieren, wenn sie durch die mindestens eine Aussparung (110) hindurch geführt ist.

4. Elektrodenkontakt (1) nach einem der vorstehenden Ansprüche, **dadurch gekennzeichnet, dass** der Auslegerabschnitt (11) mindestens eine Befestigungsfläche (115) aufweist, auf welcher ein Abschnitt der mindestens einen elektrischen Zuleitung (210) zu liegen kommt, wenn sie durch die mindestens eine Aussparung (110) hindurch geführt ist.

5. Elektrodenkontakt (1) nach Anspruch 4, **dadurch gekennzeichnet, dass** die mindestens eine Befestigungsfläche (115) für eine stoffschlüssige Befestigung des Abschnitts der mindestens einen elektrischen Zuleitung (210), insbesondere mittels Schweißen, geeignet ist.

6. Elektrodenkontakt (1) nach einem der vorstehenden Ansprüche, **dadurch gekennzeichnet, dass** die mindestens eine Aussparung (110) lochförmig ist.

7. Elektrodenleitung (2) für eine Neurostimulationsanwendung, wobei die Elektrodenleitung (2) ein flaches Paddle (22) mit einer Anzahl von Elektrodenkontakten zur Kontaktierung von menschlichem oder tierischem neuronalem Gewebe umfasst,
**dadurch gekennzeichnet, dass**
die Anzahl von Elektrodenkontakten mindestens einen Elektrodenkontakt (1) nach einem der Ansprüche 1 bis 6 umfasst.

8. Elektrodenleitung (2) nach Anspruch 7, wobei die Elektrodenleitung (2) mindestens eine elektrische Zuleitung (210) umfasst, **dadurch gekennzeichnet, dass** die mindestens eine elektrische Zuleitung (210) an dem Auslegerabschnitt (11) des mindestens einen Elektrodenkontakts (1) befestigt ist.

9. Elektrodenleitung (2) nach Anspruch 8, **dadurch gekennzeichnet, dass** die mindestens eine elektrische Zuleitung (210) an dem Auslegerabschnitt (11) des mindestens einen Elektrodenkontakts (1) stoffschlüssig, insbesondere mittels Schweißen, befestigt ist.

10. Elektrodenleitung (2) nach einem der Ansprüche 7 bis 9, wobei die Elektrodenleitung (2) mindestens eine elektrische Zuleitung (210) umfasst, **dadurch gekennzeichnet, dass** die mindestens eine elektrische Zuleitung (210) den Auslegerabschnitt (11) des mindestens einen Elektrodenkontakts (1) elektrisch kontaktiert.

11. Verfahren zum Anschließen mindestens eines Elektrodenkontakts (1) an mindestens eine elektrische Zuleitung (210),
**gekennzeichnet durch**
- Bereitstellen mindestens eines Elektrodenkontakts (1) nach einem der Ansprüche 1 bis 6; und
- Befestigen der mindestens einen elektrischen Zuleitung (210) an dem Auslegerabschnitt (11) des mindestens einen Elektrodenkontakts (1) derart, dass die elektrische Zuleitung (210) sich durch die mindestens eine Aussparung (110) hindurch erstreckt.

12. Verfahren nach Anspruch 11, **dadurch gekennzeichnet, dass** die mindestens eine elektrische Zuleitung stoffschlüssig, insbesondere mittels Schweißen, an dem Auslegerabschnitt (11) befestigt wird.

13. Verfahren nach Anspruch 11 oder 12, wobei die mindestens eine Aussparung (110) schlitzförmig ist und einen Bereich des Auslegerabschnitts (11) in mehrere Stege (111, 112, 113) unterteilt, **dadurch gekennzeichnet, dass** das Verfahren umfasst:
- Einlegen eines Abschnitts der elektrischen Zuleitung (210) in die mindestens eine schlitzförmige Aussparung (110);
- Einklemmen des Abschnitts der elektrischen Zuleitung durch Verformen mindestens eines der Stege (111, 112).

14. Verfahren nach einem der Ansprüche 11 bis 13, wobei mehrere derartige Elektrodenkontakte (1) an eine oder mehrere elektrische Zuleitungen (210) angeschlossen werden **dadurch gekennzeichnet, dass**
- die elektrische Zuleitung (210) oder die elektrischen Zuleitungen (210) zunächst vorläufig an den jeweiligen Auslegerabschnitten (11) der Elektrodenkontakte (1) befestigt werden, derart, dass sie sich durch die Aussparungen (110) in den Auslegerabschnitten (11) hindurch erstrecken;
- die Positionierung einer oder mehrerer der elektrischen Zuleitungen (210) relativ zu den Auslegerabschnitten (11) danach optional angepasst wird; und
- die elektrische Zuleitung (210) oder die elektrischen Zuleitungen (210) danach, insbesondere mittels Schweißen, in einer jeweiligen finalen Position an den Auslegerabschnitten (11) befestigt werden.

## Claims

1. An electrode contact (1) for an electrode lead (2) for neurostimulation, wherein the electrode contact (1) has a contact body (10) with a contact surface (100) for contacting human or animal neural tissue, and
an arm portion (11) for contacting at least one electrical supply lead (210), wherein the arm portion (11) extends from the contact body (10), laterally in a first spatial direction (Y) that runs parallel to a main plane of extent (XY) of the contact surface (100), and wherein the arm portion (11) has at least one cut-out (110) for guiding through the at least one electrical supply lead (210),
**characterised in that**
the at least one cut-out (110) is slot-shaped and a region of the arm portion (11) is divided by the slot-shaped cut-out (110) into a plurality of webs.

2. The electrode contact (1) according to claim 1, **characterised in that** a maximum height of the contact body (100) in a third spatial direction (Z) perpendicular to the main plane of extent (XY) is less than
- a largest width of the contact body (100) in the first spatial direction (Y), and/or
- a largest width of the contact body (100) in a second spatial direction (X) parallel to the main plane of extent (XY).

3. The electrode contact (1) according to any one of the preceding claims, **characterised in that** the arm portion (11) is designed to fix the at least one electrical supply lead (210) in a force-fitting manner when said lead is guided through the at least one cut-out (110).

4. The electrode contact (1) according to any one of the preceding claims, **characterised in that** the arm portion (11) has at least one attachment surface (115), on which a portion of the at least one electrical supply lead (210) comes to rest when said lead is guided through the at least one cut-out (110).

5. The electrode contact (1) according to claim 4, **characterised in that** the at least one attachment surface (115) is suitable for integrally bonded attachment of the portion of the at least one electrical supply lead (210), in particular by means of welding.

6. The electrode contact (1) according to any one of the preceding claims, **characterised in that** the at least one cut-out (110) is hole-shaped.

7. An electrode lead (2) for a neurostimulation application, wherein the electrode lead (2) comprises a flat paddle (22) with a number of electrode contacts for contacting human or animal neural tissue,
**characterised in that**
the number of electrode contacts comprises at least one electrode contact (1) according to any one of claims 1 to 6.

8. The electrode lead (2) according to claim 7, wherein the electrode lead (2) comprises at least one electrical supply lead (210), **characterised in that** the at least one electrical supply lead (210) is attached to the arm portion (11) of the at least one electrode contact (1).

9. The electrode lead (2) according to claim 8, **characterised in that** the at least one electrical supply lead (210) is attached to the arm portion (11) of the at least one electrode contact (1) in an integrally bonded manner, in particular by means of welding.

10. The electrode lead according to any one of claims 7 to 9, wherein the electrode lead (2) comprises at least one electrical supply lead (210), **characterised in that** the at least one electrical supply lead (210) electrically contacts the arm portion (11) of the at least one electrode contact (1).

11. A method for connecting at least one electrode contact (1) to at least one electrical supply lead (210),
**characterised by**
- providing at least one electrode contact (1) according to any one of claims 1 to 6; and
- attaching the at least one electrical supply lead (210) to the arm portion (11) of the at least one electrode contact (1) in such a way that the electrical supply lead (210) extends through the at least one cut-out (110).

12. The method according to claim 11, **characterised in that** the at least one electrical supply lead is attached to the arm portion (11) in an integrally bonded manner, in particular by means of welding.

13. The method according to claim 11 or 12, wherein the at least one cut-out (110) is slot-shaped and divides a region of the arm portion (11) into a plurality of webs (111, 112, 113), **characterised in that** the method comprises:
- inserting a portion of the electrical supply lead (210) into the at least one slot-shaped cut-out (110); and
- clamping the portion of the electrical supply lead by deforming at least one of the webs (111, 112).

14. The method according to any one of claims 11 to 13, wherein a plurality of such electrode contacts (1) are connected to one or more electrical supply leads (210), **characterised in that**
- the electrical supply lead (210) or the electrical supply leads (210) are initially temporarily attached to the respective arm portions (11) of the electrode contacts (1) in such a way as to extend through the cut-outs (110) in the arm portions (11);
- thereafter, the position of one or more of the electrical supply leads (210) relative to the arm portions is optionally adapted; and
- thereafter, the electrical supply lead (210) or the electrical supply leads (210) are attached to the arm portions (11) in the respective final position, in particular by means of welding.

## Revendications

1. Contact d'électrode (1) pour un câble d'électrode (2) pour neurostimulation, dans lequel le contact d'électrode (1) a un corps de contact (10) avec une surface de contact (100) destinée à être mise en contact avec un tissu neuronal humain ou animal, et
une partie de bras (11) destinée à être mise en contact avec au moins un câble d'alimentation électrique (210), dans lequel la partie de bras (11) s'étend depuis le corps de contact (10), latéralement dans une première direction spatiale (Y) qui court parallèlement à un plan principal d'extension (XY) de la surface de contact (100), et dans lequel la partie de bras (11) a au moins une découpe (110) pour guider l'au moins un câble d'alimentation électrique (210) à travers,
**caractérisé en ce que**
l'au moins une découpe (110) est en forme de fente et une région de la partie de bras (11) est divisée par la découpe en forme de fente (110) en une pluralité de toiles.

2. Contact d'électrode (1) selon la revendication 1, **caractérisé en ce que** une hauteur maximale du corps de contact (100) dans une troisième direction spatiale (Z) perpendiculaire au plan principal d'extension (XY) est inférieure à
- une largeur la plus grande du corps de contact (100) dans la première direction spatiale (Y), et/ou
- une largeur la plus grande du corps de contact (100) dans une seconde direction spatiale (X) parallèle au plan principal d'extension (XY).

3. Contact d'électrode (1) selon l'une quelconque des revendications précédentes, **caractérisé en ce que** la partie de bras (11) est conçue pour fixer l'au moins un câble d'alimentation électrique (210) d'une manière engagée en frottement lorsque ledit câble est guidé à travers l'au moins une découpe (110).

4. Contact d'électrode (1) selon l'une quelconque des revendications précédentes, **caractérisé en ce que** la partie de bras (11) a au moins une surface de liaison (115), sur laquelle une partie de l'au moins un câble d'alimentation électrique (210) vient reposer lorsque ledit câble est guidé à travers l'au moins une découpe (110).

5. Contact d'électrode (1) selon la revendication 4, **caractérisé en ce que** l'au moins une surface de liaison (115) est adaptée pour une liaison intégralement collée de la partie de l'au moins un câble d'alimentation électrique (210), en particulier au moyen d'un soudage.

6. Contact d'électrode (1) selon l'une quelconque des revendications précédentes, **caractérisé en ce que** l'au moins une découpe (110) est en forme de trou.

7. Câble d'électrode (2) pour une application de neurostimulation, dans lequel le câble d'électrode (2) comprend une spatule plate (22) avec un certain nombre de contacts d'électrode destinés à être mis en contact avec un tissu neuronal humain ou animal,
**caractérisé en ce que**
le certain nombre de contacts d'électrode comprend au moins un contact d'électrode (1) selon l'une quelconque des revendications 1 à 6.

8. Câble d'électrode (2) selon la revendication 7, dans lequel le câble d'électrode (2) comprend au moins un câble d'alimentation électrique (210), **caractérisé en ce que** l'au moins un câble d'alimentation électrique (210) est lié à la partie de bras (11) de l'au moins un contact d'électrode (1).

9. Câble d'électrode (2) selon la revendication 8, **caractérisé en ce que** l'au moins un câble d'alimentation électrique (210) est lié à la partie de bras (11) de l'au moins un contact d'électrode (1) d'une manière intégralement collée, en particulier au moyen d'un soudage.

10. Câble d'électrode selon l'une quelconque des revendications 7 à 9, dans lequel le câble d'électrode (2) comprend au moins un câble d'alimentation électrique (210), **caractérisé en ce que** l'au moins un câble d'alimentation électrique (210) entre en contact électrique avec la partie de bras (11) de l'au moins un contact d'électrode (1).

11. Procédé de connexion d'au moins un contact d'électrode (1) à au moins un câble d'alimentation électrique (210),
**caractérisé par**
- fournir au moins un contact d'électrode (1) selon l'une quelconque des revendications 1 à 6 ; et
- lier l'au moins un câble d'alimentation électrique (210) à la partie de bras (11) de l'au moins un contact d'électrode (1) d'une façon telle que le câble d'alimentation électrique (210) s'étend à travers l'au moins une découpe (110).

12. Procédé selon la revendication 11, **caractérisé en ce que** l'au moins un câble d'alimentation électrique est lié à la partie de bras (11) d'une manière intégralement collée, en particulier au moyen d'un soudage.

13. Procédé selon la revendication 11 ou 12, dans lequel l'au moins une découpe (110) est en forme de fente et divise une région de la partie de bras (11) en une pluralité de toiles (111, 112, 113), **caractérisé en ce que** le procédé comprend :
- l'insertion d'une partie du câble d'alimentation électrique (210) dans l'au moins une découpe en forme de fente (110) ; et
- l'enclenchement de la partie du câble d'alimentation électrique en déformant au moins l'une des toiles (111, 112).

14. Procédé selon l'une quelconque des revendications 11 à 13, dans lequel une pluralité de tels contacts d'électrodes (1) sont connectés à un ou plusieurs câbles d'alimentation électrique (210), **caractérisé en ce que**
- le câble d'alimentation électrique (210) ou les câbles d'alimentation électrique (210) sont initialement temporairement liés aux parties de bras (11) respectives des contacts d'électrode (1) d'une façon telle qu'ils s'étendent à travers les découpes (110) dans les parties de bras (11) ;
- ensuite, la position d'un ou de plusieurs des câbles d'alimentation électrique (210) par rapport aux parties de bras est éventuellement adaptée ; et
- ensuite, le câble d'alimentation électrique (210) ou les câbles d'alimentation électrique (210) sont liés aux parties de bras (11) dans la position finale respective, en particulier au moyen d'un soudage.
